(19) Europäisches Patentamt
European Patent Office
Office européen des brevets

(11) Numéro de publication : **0 403 331 B1**

(12) # FASCICULE DE BREVET EUROPEEN

(45) Date de publication du fascicule du brevet : **24.03.93 Bulletin 93/12**

(51) Int. Cl.[5] : **C07C 22/04,** C07C 17/38

(21) Numéro de dépôt : **90401490.9**

(22) Date de dépôt : **01.06.90**

(54) **Procédé de séparation de deux isomères, son application à la purification du phényl-1 bromo-2 éthane.**

(30) Priorité : **13.06.89 FR 8907800**

(43) Date de publication de la demande : **19.12.90 Bulletin 90/51**

(45) Mention de la délivrance du brevet : **24.03.93 Bulletin 93/12**

(84) Etats contractants désignés : **AT BE CH DE DK ES FR GB GR IT LI LU NL SE**

(56) Documents cités :
**US-A- 3 058 892**
**US-A- 3 453 339**

(73) Titulaire : **ELF ATOCHEM S.A.**
**4 & 8, Cours Michelet La Défense 10**
**F-92800 Puteaux (FR)**

(72) Inventeur : **Commandeur, Raymond**
**Le Rocher, Avenue de Vénaria**
**F-38220 Vizille (FR)**
Inventeur : **Drivon, Gilles**
**3 Rue Joanny Courbière**
**F-69850 Saint-Martin En Haut (FR)**
Inventeur : **Ghenassia, Elie**
**20 Avenue Albert Ier de Belgique**
**F-38100 Grenoble (FR)**

**EP 0 403 331 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de séparation de deux isomères et son application à la purification du phényl-1 bromo-2 éthane.

Le phényl-1 bromo-2 éthane ($C_6H_5$-$CH_2$-$CH_2Br$), ou bromure de phénéthyle est un intermédiaire de synthèse organique et il est préparé par l'hydrobromation radicalaire du styrène par l'acide bromhydrique gazeux :

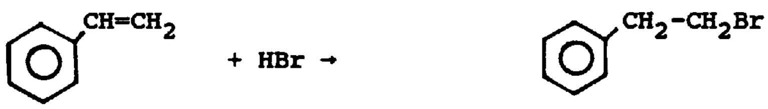


L'addition de type anti-MARKOVNIKOV est favorisée par l'introduction simultanée d'air et d'HBr gazeux, dans le styrène en solution dans un solvant inerte peu polaire comme le tétrachlorure de carbone. La réaction qui n'est jamais totalement sélective est concurrencée par l'addition de type MARKOVNIKOV :

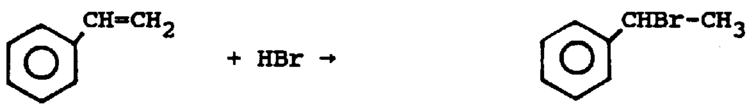


dont l'importance croît avec la concentration du styrène dans le $CCl_4$. Cette réaction est par ailleurs favorisée par la présence d'impuretés métalliques comme le fer.

La séparation du bromure de phénéthyle d'avec son isomère le bromo-1 phényl-1 éthane est difficile par distillation vu le faible écart des points d'ébullition : 217°C pour le bromure de phénéthyle, 203°C pour le bromo-1 phényl-1 éthane.

L'art antérieur US 3 058 892, propose de traiter le mélange de bromure de phénéthyle et de son isomère par l'oxyde de zinc entre 100 et 150°C pour provoquer la décomposition sélective de l'isomère. Il est recommandé de minimiser la température et le temps de réaction pour éviter la décomposition du bromure de phénéthyle.

On a maintenant trouvé un autre procédé qui est exactement le contraire du précédent puisqu'il consiste à condenser sélectivement le bromo-1 phényl-1 éthane avec un autre produit pour en faire un produit lourd facile à séparer par distillation.

Un autre avantage de l'invention est qu'on opère à plus faible température que dans l'art antérieur. Au cours de ses recherches, la demanderesse a découvert un procédé pour séparer de façon sélective de nombreux couples d'isomères.

La présente invention est donc un procédé de séparation de deux isomères de formule :

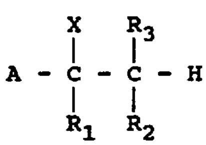 (I) et 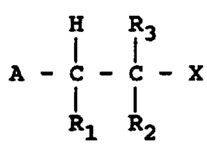 (II)

dans lesquelle A désigne un groupe aryle pouvant être substitué ; X désigne un halogène ; $R_1$ désigne $X_1$, l'hydrogène ou un groupe alkyl ; $R_2$ ; désigne $X_2$, H ou un groupe alkyl ; $X_1$ et $X_2$ désignent un halogène ; $R_3$ désigne l'hydrogène ou un groupe alkyl, caractérisé en ce que :

a) on condense l'isomère (I) avec un produit portant au moins un hydrogène arylique et en présence d'un catalyseur de FRIEDEL ET CRAFTS,

b) puis on sépare le produit de condensation obtenu d'avec l'isomère (II).

Ces deux isomères résultent par exemple de l'addition d'un halogénure d'hydrogène sur le produit de formule :

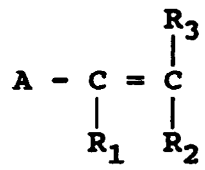

A, $R_1$, $R_2$ et $R_3$ ayant les mêmes significations que précédemment.

Avantageusement A désigne un radical dérivé du benzène, du biphényle, du naphtalène ou de l'anthracène. A peut être substitué par des halogènes, des groupements $NO_2$, CN, alkyls ou alkoxy.

Avantageusement, X, $X_1$ et $X_2$ représentent le chlore ou le brome.

$R_1$, $R_2$ et $R_3$ désignent aussi des groupes alkyls, ces groupes alkyls peuvent avoir jusqu'à 4 atomes de carbone. De préférence les isomères (I) et (II) sont tels que A est le phényl pouvant être substitué, $R_1$ et $R_2$ sont des alkyls ayant jusqu'à 4 atomes de carbone et $R_3$ est l'hydrogène.

L'isomère (I) est condensé avec un produit portant au moins un hydrogène arylique. Ce produit peut être quelconque pourvu qu'il porte un hydrogène suffisamment mobile et qu'il se couple avec l'isomère (I) avec élimination de HX. On utilise avantageusement les hydrocarbures aromatiques, les éthers ayant une partie aromatique, le phénol. Parmi les hydrocarbures aromatiques, on peut citer le benzène, le toluène, l'éthylbenzène, le cumène, les xylènes. Parmi les éthers, on peut citer le phényl méthyl éther ($C_6H_5$-O-CH3). On ne sortirait pas du cadre de l'invention en utilisant un mélange d'hydrocarbures aromatiques, d'éthers, ou toute combinaison de ces produits.

Les isomères (I) et (II) peuvent être seuls ou dans un mélange de solvant. Le catalyseur de FRIEDEL et CRAFTS est connu en lui-même. On désigne ainsi les acides de LEWIS (halogénures métalliques..), les acides protoniques, les zéolites. Parmi les halogénures métalliques on peut citer les chlorure ferrique, le chlorure d'aluminium. Parmi les acides protoniques, on peut citer les oxyacides tels que l'acide sulfurique, l'acide paratoluène sulfonique ; parmi les zéolites on peut citer les faujasites X ou Y décationisées.

A titre d'exemple dans un mélange de bromure de phénéthyle (C6H5-CH2-CH2Br) et de phényl-1 bromo-1 éthane (C6H5-CHBr-CH3) seul ce dernier se condense avec du métaxylène pour former du phénylxylyléthane selon la réaction :

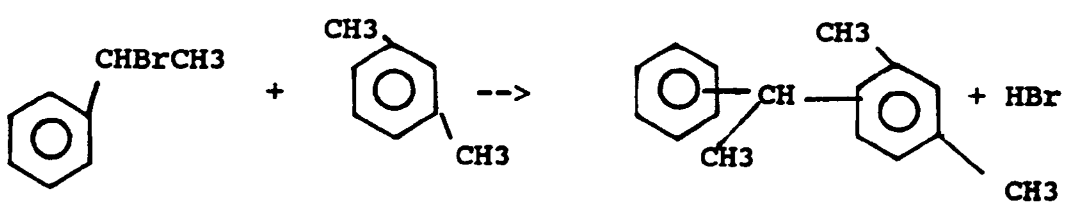

Le phénylxylyléthane ainsi formé a un point d'ébullition élevé (supérieur à 300°C) et ne pose donc pas de problème de séparation par distillation d'avec le bromure de phénéthyle (point d'ébullition 217°C). La quantité de catalyseur peut varier dans de larges limites, on peut utiliser par exemple 0,5 à 2 g $FeCl_3$ par Kg de bromure de phénéthyle. La température peut varier dans de larges limites, avantageusement on se place dans une zone de température ou les isomères et le produit avec lequel on condense sont liquides, on préfère opérer entre 50 et 100°C. La pression est aussi choisie pour pouvoir opérer la condensation à la pression atmosphérique ou à une pression n'excédant pas quelques bars.

La durée de réaction est comprise entre quelques minutes et 2 ou 3 heures ; cette durée décroît avec la température.

Si les isomères sont déjà dans un solvant tel qu'un hydrocarbure aromatique, un éther ayant une partie aromatique ou du phénol, il suffit pour réaliser l'invention d'ajouter le catalyseur dans ce mélange.

Après l'étape a du procédé, c'est-à-dire la condensation, on peut laver le mélange réactionnel avec une solution aqueuse d'acide chlorhydrique. On effectue ensuite l'étape b, c'est-à-dire la séparation pour récupérer l'isomère II. On peut utiliser tout moyen, mais il est avantageux de distiller.

Les exemples suivants illustrent l'invention.

**EXEMPLE 1**

On opère dans l'appareillage suivant :

- Erlenmeyer de 1 litre avec un piquage,
- Gaine thermométrique avec thermocouple,
- Couverture d'azote,
- Réfrigérant ascendant à eau,
- Barboteur à eau.

On charge dans l'Erlenmeyer 370 g de bromure de phénéthyle brut dont la composition est indiquée dans le tableau I, ainsi que 212 g de mélange d'isomères de xylène (produit commercial riche en isomère méta). On ajoute ensuite 0,5 g de chlorure ferrique anhydre et l'ensemble est porté à 60°C sous balayage d'azote. Le courant gazeux passe dans le barboteur a eau et l'acide bromhydrique formé est dosé en continu par la soude en présence de phénolphtaléine. La quantité d'HBr dégagée (0,165 mole) correspond sensiblement à la quantité de phényl-1 bromo-1 éthane (0,153 mole).

Par ailleurs, l'analyse chromatographique effectuée sur un prélèvement au bout de 5 H 10 de réaction (voir tableau I) met en évidence la disparition totale du phényl-1 bromo-1 éthane.

La même analyse effectuée au bout de 6 H 40 montre qu'il n'y a pratiquement pas d'évolution du milieu. Il n'y a pas de diminution de la teneur en bromure de phénéthyle, c'est-à-dire que le produit est stable dans ces conditions (les échantillons sont lavés immédiatement à l'eau + HCl 10 % avant d'effectuer l'analyse).

En fin d'essai, la totalité du milieu réactionnel est lavée à l'acide chlorhydrique 10 % et ensuite à l'eau. La phase organique est ensuite placée dans un ballon de distillation surmonté d'une colonne de cinq plateaux, puis soumise à une opération de séchage azéotropique sous vide de manière à maintenir la température dans le ballon vers 80°C. Après fin de séchage, on installe une tête de reflux sur la colonne à distiller et le contenu du ballon est distillé sous vide progressif avec un léger reflux. La fraction de distillation passant entre 101 et 103°C sous 15 mm de mercure (voir analyse sur le tableau I) représente 90 % de la quantité de bromure de phénéthyle présent dans le produit de départ. La température du pied est de 170°C en fin de distillation et nous n'avons pas constaté de décomposition.

Le bromure de phénéthyle ainsi obtenu (incolore) a une pureté supérieure à 98,5 %. Il pourrait avoir une pureté supérieure en affinant la distillation. En effet, compte tenu de son point d'ébullition nettement plus élevé, le dibromo-1,2-éthylbenzène pourrait être facilement séparé.

Nous constatons, d'autre part, à l'examen des résultats du tableau, la disparition ou la diminution d'un certain nombre de produits autres que le phényl-1 bromo-1 éthane (pics 7, 12, 14). Ces produits, non identifiés, possèdent vraisemblablement un atome de brome réactif qui pourrait être facteur d'instabilité lors d'une distillation de bromure de phénéthyle brut, n'ayant pas subi le traitement que nous venons de décrire. Le phényl-1 bromo-1 éthane s'est condensé avec le xylène pour former du phénylxylyléthane.

**TABLEAU I (résultats de l'exemple 1)**

| | Bromure de phénéthyle brut | Xylènes | Mélange bromure de phénéthyle- + xylènes avant réaction | Prélèvements après 5 h 10 de réaction | Prélèvements après 6 h 40 de réaction | Bromure de phénéthyle distillé | Résidu de distillation |
|---|---|---|---|---|---|---|---|
| 1 | 0,45 | 0,49 | 0,62 | 0,52 | 0,57 | 0,57 | - |
| 2 | 0,45 | - | 0,25 | 0,09 | 0,09 | - | - |
| 3 | - | 0,98 | 0,77 | 0,65 | 0,66 | 0,14 | - |
| 4 (Ethylbenzène) | - | 21,61 | 8,57 | 10,69 | 10,66 | - | - |
| 5 (m+p.xylène) | 0,39 | 69,49 | 36,77 | 30,10 | 29,64 | - | - |
| 6 (orthoxylène) | - | 7,16 | 3,73 | 4,47 | 4,38 | - | - |
| 7 | 0,30 | - | 0,22 | - | - | x | - |
| 8 (bromo-1 phényl-1 éthane) | 7,63 | - | 3,29 | - | - | - | - |
| 9 (bromure de phénéthyle | 80,29 | - | 41,06 | 41,86 | 41,99 | 98,76 | 0,98 |
| 10 (dibromo-1,2 éthylbenzène) | 7,51 | - | 3,13 | 3,46 | 3,40 | 0,53 | 22,44 |
| 11 (phénylxylyléthane) | - | - | 0,26 | 4,77 | 4,79 | - | 37,64 |
| 12 | 0,81 | - | 0,40 | x | x | - | x |
| 13 | 0,75 | - | 0,33 | 0,56 | 0,57 | - | 4,49 |
| 14 | 0,83 | - | 0,28 | x | - | - | 0,48 |
| 15 | 0,58 | - | 0,32 | 0,32 | 0,32 | - | Σ 4,41 |
| 16 | - | - | - | 1,24 | 1,45 | - | 12,57 |
| 17 | - | - | - | 1,27 | 1,48 | - | 12,51 |
| 18 | - | - | - | x | x | - | 3,69 |

"x" désigne des traces
Les numéros en 1ère colonne indiquent l'ordre de sortie sur le chromatogramme
Les produits 1 à 3 sont des légers, les produits 12 à 18 sont des lourds
Les compositions sont en pourcentage surface des produits élués en chromatographie
"10" s'appelle aussi dibromo-1,2 phényl-1 éthane.

**EXEMPLE 2**

Dans le même appareillage que dans l'exemple 1 on traite un mélange de :

- 72,6 % d'éthylbenzène,
- 25,8 % de chloro-1 phényl-1 éthane,
- 1,6 % de chloro-2 phényl-1 éthane.

Le mélange est coulé en 1 h 30 dans 12,6 moles d'éthylbenzène, a 70°C, contenant 1,64 g de $FeCl_3$ anhydre.

Le milieu réactionnel est ensuite maintenu pendant encore 3 h à 70°C. L'analyse chromatographique sur divers échantillons, après fin de coulage jusqu'à l'arrêt de l'essai, met en évidence :

- la disparition très rapide du chloro-1 phényl-1 éthane
- l'absence de réaction du chloro-2 phényl-1 éthane.

**EXEMPLE 3**

Dans un appareillage identique à celui de l'exemple 1, on charge 370 g de bromure de phénéthyle brut dont la composition est indiquée dans le tableau II ainsi que 85 g de toluène. On introduit ensuite 0,12 g de chlorure ferrique anhydre et l'ensemble est porté à 85-125°C sous balayage d'azote. Le courant gazeux passe dans le barboteur à eau et l'acide bromhydrique formé est dosé en continu. La quantité d'HBr dégagé en fin de réaction est de 0,451 mole.

Par ailleurs, l'analyse chromatographique (voir tableau II) met en évidence la disparition très rapide du bromo-1 phényl-1 éthane ainsi que la disparition progressive du dibromo-1,2 éthylbenzène (ou dibromo-1,2 phényl-1 éthane). Par contre la teneur en bromure de phénéthyle n'évolue pas pendant toute la durée du traitement. Le milieu réactionnel élaboré de la même façon que dans l'exemple 1 conduit après distillation au bromure de phénéthyle de pureté supérieure a 99,5 % avec un rendement de 90 % par rapport à la quantité engagée au départ.

**TABLEAU II**

| | EVOLUTION DE LA COMPOSITION | | | | |
|---|---|---|---|---|---|
| | Bromure de phénéthyle brut | Après 1 h de réaction | Après 2 h de réaction | Après 4 h 45 de réaction | Après 5 h 45 de réaction |
| 1) | 0,42 | 0,39 | 0,38 | 0,34 | 0,49 |
| 2) | - | 0,14 | 0,14 | - | - |
| 3) toluène | | 22 | 22 | 20,55 | 21,04 |
| 4) Bromo-1 phényl-1 éthane | 7,59 | - | - | - | - |
| 5) Bromure de phénéthyle | 80,66 | 60,36 | 61,58 | 64,68 | 67,38 |
| 6) Dibromo-1,2 éthylbenzène | 7,77 | 4,76 | 3,41 | 1,54 | X |
| 7) Phényltolyléthane | | 4,33 | 4,33 | 4,45 | 4,40 |
| 8) | 0,36 | 0,65 | 0,59 | 0,58 | 0,65 |
| 9) | 0,38 | 0,74 | 0,62 | 0,57 | 0,56 |
| 10) | 0,83 | - | - | - | - |
| 11) | | X | 0,31 | 0,41 | - |
| 12) | | 0,54 | 0,91 | 0,94 | - |
| 13) | 0,72 | 1 | 0,88 | 1,03 | 1,07 |
| 14) | | 0,34 | 0,40 | 0,70 | 1,11 |
| 15) | | 0,23 | 0,18 | X | X |
| 16) | | 2,84 | 2,73 | 2,76 | 2,53 |

Les compositions sont données en pourcentage surface des produits élués en chromatographie
- signifie "non détecté"
X signifie "traces"

## Revendications

**1.** Procédé de séparation de deux isomères de formule :

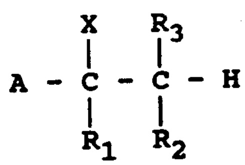 (I) et 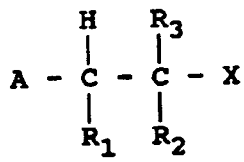 (II)

dans lesquelle A désigne un groupe aryle pouvant être substitué ; X désigne un halogène ; $R_1$ désigne $X_1$, l'hydrogène ou un groupe alkyl ; $R_2$ désigne $X_2$, H ou un groupe alkyl ; $X_1$ et $X_2$ désignent un halogène; $R_3$ désigne l'hydrogène ou un groupe alkyl, caractérisé en ce que :

a) on condense l'isomère (I) avec un produit portant au moins un hydrogène arylique et en présence d'un catalyseur de FRIEDEL ET CRAFTS,

b) puis on sépare le produit de condensation obtenu d'avec l'isomère (II).

**2.** Procédé selon la revendication 1, caractérisé en ce que A est le phényl pouvant être substitué, $R_1$ et $R_2$ sont des alkyls ayant jusqu'à 4 atomes de carbone, $R_3$ est l'hydrogène et le halogène X est du chlore ou du brome.

**3.** Procédé selon la revendication 1 ou 2, caractérisé en ce que le produit portant un hydrogène arylique est choisi parmi les hydrocarbures aromatiques, les éthers ayant une partie aromatique et le phénol.

**4.** Procédé selon l'une des revendications 1 à 3, caractérisé en ce que le catalyseur de FRIEDEL et CRAFTS est du chlorure ferrique.

**5.** Procédé selon l'une des revendications 2 à 4, caractérisé en ce qu'on l'applique à la purification du bromure de phénéthyle.

## Patentansprüche

**1.** Verfahren zur Trennung von zwei Isomeren der Formel:

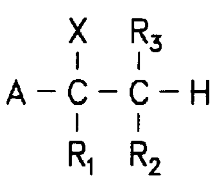 (I) und 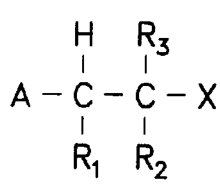 (II)

wobei A eine Arylgruppe darstellt, die substituiert sein kann; X ein Halogenatom darstellt; $R_1$ ein Wasserstoffatom, eine Alkylgruppe oder $X_1$ bezeichnet; $R_2$ ein Wasserstoffatom, eine Alkylgruppe oder $X_2$ bezeichnet; $X_1$ und $X_2$ Halogenatome darstellen; $R_3$ ein Wasserstoffatom oder eine Alkylgruppe bezeichnet und das Verfahren dadurch gekennzeichnet ist, daß man

a) das Isomere (I) in Gegenwart eines Friedel-Craft's-Katalysators mit einem Produkt kondensiert, welches mindestens ein arylisches Wasserstoffatom trägt und

b) anschließend das erhaltene Kondensationsprodukt vom Isomeren (II) abtrennt.

**2.** Verfahren nach Anspruch 1, dadurch gekennzeichnet, daß A ein Phenylrest ist, der substituiert sein kann,

$R_1$ und $R_2$ Alkylreste mit bis zu 4 Kohlenstoffatomen sind, $R_3$ ein Wasserstoffatom ist und das Halogen X ein Chlor- oder Bromatom ist.

3. Verfahren nach Anspruch 1 oder 2, dadurch gekennzeichnet, daß das Produkt, welches ein arylisches Wasserstoffatom enthält, ein aromatischer Kohlenwasserstoff, ein Ether mit aromatischer Teilstruktur oder Phenol sein kann.

4. Verfahren nach einem der Ansprüche 1 bis 3, dadurch gekennzeichnet, daß der Friedel-Craft's-Katalysator Eisenchlorid ist.

5. Verfahren nach einem der Ansprüche 2 bis 4, dadurch gekennzeichnet, daß es zur Reinigung von Phenethylbromid angewendet wird.

## Claims

1. Process for the separation of two isomers of formula:

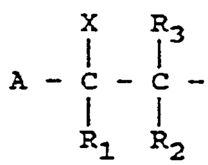 (I) and 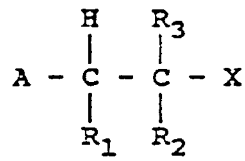 (II)

in which A denotes an aryl group, which may be substituted; X denotes a halogen; $R_1$ denotes $X_1$, hydrogen or an alkyl group; $R_2$ denotes $X_2$, H or an alkyl group; $X_1$ and $X_2$ denote a halogen; and $R_3$ denotes hydrogen or an alkyl group, characterised in that:

a) the isomer (I) is subjected to a condensation reaction with a product carrying at least one aryl hydrogen and in the presence of a Friedel-Crafts catalyst, and

b) the condensation product obtained is then separated from the isomer (II).

2. Process according to claim 1, characterised in that A is phenyl, which may be substituted, $R_1$ and $R_2$ are alkyls having up to 4 carbon atoms, $R_3$ is hydrogen and the halogen X is chlorine or bromine.

3. Process according to claim 1 or 2, characterised in that the product carrying an aryl hydrogen is chosen from aromatic hydrocarbons, ethers having an aromatic part and phenol.

4. Process according to one of claims 1 to 3, characterised in that the Friedel-Crafts catalyst is ferric chloride.

5. Process according to one of claims 2 to 4, characterised in that it is applied to the purification of phenethyl bromide.